# EUROPEAN PATENT APPLICATION

(11) **EP 4 248 907 A1**
(43) Date of publication of application: **27.09.2023**
(21) Application number: 22382274.3
(22) Date of filing: 24.03.2022
(51) Int. Cl.: A61C 8/00, A61C 9/00, A61B 90/00

(54) **SCAN ABUTMENT FOR DENTAL IMPLANTS, METHOD FOR CREATING A MODEL, AND USE OF THE SCAN ABUTMENT**

(71) Applicant: Implant Protesis Dental 2004 S.L., 08303 Mataró (Barcelona) (ES)
(72) Inventor: Miguel Ángel, Nieves Pérez, 08302 Mataró (ES)
(74) Representative: Curell Suñol S.L.P.

(57) **Abstract**

The present invention relates to a scan abutment (1) for dental implants comprising a coupling base (2) for being coupled to a dental implant, a rod (3) extending from the coupling base (2) along an axis (X), and a scan head (4) formed at one end of the rod (3). The scan head (4) comprises at least one singular area (5, 6) that can be recognized by an optical scanner system for determining a position and an orientation of the scan abutment (1). The rod (3) comprises a shaft (7) with an outer surface (8) and retaining projections (9) extending from the shaft (7), radially with respect to the axis (X), from the outer surface (8) of the shaft (7). The invention also comprises a method for forming a model of an upper or lower jaw region using the scan abutment (1).

## Description

### Field of the invention

The invention is comprised in the field of dental implantology.

More specifically, the invention relates to a scan abutment for dental implants of the type suitable for being removably fixed to a dental implant implanted in an upper jaw bone or a lower jaw bone of a mouth, said scan abutment being a monoblock piece comprising a coupling base for coupling said scan abutment, directly or through an intermediate piece, to the dental implant, a rod extending from the coupling base along an axis, and a scan head formed at one end of said rod opposite said coupling base, said scan head comprising on its outer surface at least one singular area that can be recognized by an optical scanner system for determining a position and an orientation of said scan abutment.

The invention also relates to a method for creating a model of an upper or lower jaw region of a mouth using at least two scan abutments according to the invention, in which at least two dental implants are implanted in a bone of said jaw dental region, and to the use of the scan abutment to create the model.

### State of the Art

The invention is applied to methods for restoring teeth in the mouth of a patient by means of dental prostheses which are fixed to dental implants implanted in an upper jaw bone or a lower jaw bone. Dental prostheses are usually fixed to dental implants directly or through an interposed piece such as, for example, a transepithelial abutment, by means of a fixing screw. These dental prostheses are made in a laboratory and must be fitted perfectly in the patient's mouth. To make dental prostheses in the laboratory, a model which reproduces, as accurately as possible, an upper or lower jaw region of the mouth in which dental implants are implanted is used. The model contains pieces called analogs which are inserted in the body of the model and the function of which is to accurately reproduce, with the same shape and position, the accessible part of the dental implant to which the dental prosthesis is coupled. In the laboratory, dental prostheses are coupled to the analogs in the model, whereby they have exactly the same position and orientation as when they are coupled to the dental implants in the mouth.

Different methods for making the model which contains the analogs are known. A conventional method consists of making an impression of the upper or lower jaw region of the mouth in a mass of impression material and using this impression as a mold which is filled with a special plaster. A plaster model which accurately reproduces the upper or lower jaw region is thereby obtained. To place the analogs in the model such that they accurately reproduce the position and orientation of the dental implants, a piece called impression coping is screwed to each dental implant before making the impression. When the impression is made, the impression copings fixed to the dental implants are plunged into the mass of impression material. Next, the screws fixing the impression copings to the dental implants are removed and an integral assembly formed by the mass of impression material and the impression copings plunged into same is removed from the mouth. To make the plaster model using this integral assembly as a mold, an analog is fixed to each impression coping by means of a fixing screw and the integral assembly is covered with a mass of plaster which covers the analogs. When the plaster has hardened, the fixing screws are taken out and the integral assembly removed. The result is a master plaster copy containing the analogs placed in the exact same position as the corresponding dental implants in the patient's mouth. EP2368518A1 discloses an impression coping and a conventional method such as the one which has been described. The impression coping has a rod and retaining projections for retaining said impression coping in the mass of impression material.

In recent years, digital methods based on digital and 3D printing technologies, which allow manufacturing the model with a 3D printer, have been developed. In these methods, an optical scanner system is used for scanning the upper or lower jaw region of the mouth. The optical scanner system comprises an intraoral optical scanner and associated software. The reading performed by the intraoral optical scanner is sent to the laboratory and processed using software providing a data file which defines the 3D geometry of the surface of the upper or lower jaw region which has been scanned. To determine the position and the orientation of the dental implants using the optical scanner system, pieces called scan abutments are used. A scan abutment is as described above. EP2457536A2 discloses a scan abutment of this type. Scan abutments are fixed to dental implants in the mouth, such that scanning of the upper or lower jaw region is performed with these scan abutments fixed to the dental implants. The software of the optical scanner system recognizes the singular areas in the scan head of each scan abutment, and based on this recognition, deduces a position and an orientation of the scan abutment, and therefore of the corresponding dental implant. The data provided by the optical scanner system are used to manufacture the model comprising housings for receiving the analogs by means of 3D printing. In addition to being used to manufacture the model by means of 3D printing, the data provided by the optical scanner system are also used to obtain a virtual model which is used in an application for designing the dental prosthesis, particularly for selecting the pieces which must form the dental prosthesis in a digital library provided by the manufacturer.

This completely digital method is more efficient than the conventional method which consists of making a plaster mold from an impression of the mouth as described above. However, it basically has two drawbacks. The first drawback is that a model which has been printed with a 3D printer is not very precise because it is made with a photohardenable resin which, after 3D printing, still experiences slight dimensional changes since it is exposed to light. Furthermore, a 3D printer has a precision error which is not always easy to correct and is different depending on the model of printer used. The second problem is that intraoral optical scanning may be imprecise because the dentist lacks skill or knowledge, or because the intraoral scanner is not properly calibrated. Furthermore, dentists use different intraoral scanner models which have different precision errors. As a result, a mold obtained by 3D printing based on optical scanning is usually less precise than a plaster mold obtained from an impression of the mouth.

### Description of the invention

The purpose of the invention is to provide a scan abutment for dental implants of the type indicated above which can be used in both a digital method and a conventional method like the ones described above, and such that, when used in a conventional method, it allows optical scanning to be performed in an easier and more precise manner in order to determine the position and the orientation of the dental implants.

This purpose is achieved by means of a scan abutment for dental implants of the type indicated above, characterized in that the rod comprises a shaft with an outer surface and retaining projections extending from said shaft, radially with respect to the axis, from said outer surface of the shaft. The retaining projections are suitable for retaining the scan abutment in a mass of impression material into which the rod is plunged when an impression of an upper or lower jaw region of the mouth comprising said scan abutment fixed to a dental implant is taken with said impression material.

As will be seen below in the detailed description of the embodiments, the scan abutment according to the invention can advantageously be used as an impression coping to create a mold made of plaster or of another similar material from an impression of the upper or lower jaw region of the mouth in a mass of impression material, and at the same time as a scan abutment for obtaining the position and orientation of the dental implants by means of scanning with an optical scanner system.

This allows a method for creating a model of an upper or lower jaw region of a mouth to be performed using at least one scan abutment according to invention, in which at least one dental implant is implanted in a bone of said upper or lower jaw region of the mouth, said method comprising at least the following successive steps:
[a] fixing the scan abutment to the dental implant by coupling the coupling base to the dental implant;
[b] applying a mass of impression material on the upper or lower jaw region of the mouth, such that the retaining projections of the scan abutment fixed to the dental implant are plunged into the mass of impression material and the at least one singular area of the scan head of the scan abutment is outside the mass of impression material; such that there is formed in the mass of impression material an impression of the upper or lower jaw region of the mouth, in which the scan abutment is made integral with the mass of impression material by the retaining projections;
[c] releasing the scan abutment from the dental implant and removing the mass of impression material, which comprises the scan abutment integral with said mass of impression material, from the mouth;
and wherein, after step [a], preferably after step [c], scanning is performed by means of an optical scanner system which recognizes the at least one singular area of the scan head of the scan abutment; and based on the recognition of said at least one singular area (5, 6), a position and an orientation of the scan abutment (1) are determined.

Scanning can be performed in the mouth after step [a] and before steps [b] or [c]. In this case, scanning is similar to the one performed in the methods of the state of the art described above.

The difference is that, instead of first placing a scan abutment to perform scanning, removing same, and placing an impression coping to make the impression, a single piece, i.e., the scan abutment according to the invention which performs the functions of a scan abutment and an impression coping at the same time, is used in the method according to the invention.

Advantageously, scanning is performed outside the mouth after step [c], scanning the mass of impression material having the scan abutment integral with it. Greater precision is thereby obtained, since the difficulty of having to move and orient an intraoral optical scanner within the mouth is avoided. Furthermore, a fixed optical scanner, which may have greater precision than an intraoral optical scanner, can be used. Another advantage is that scanning can be performed in the laboratory, instead of by the dentist or as a complement thereof. This also contributes to increasing the precision and reliability of the data obtained by the optical scanner.

The fact that a single piece, i.e., the scan abutment, is used also contributes to increasing the precision.

The abutment according to the invention is particularly advantageous when there are at least two dental implants implanted in a bone of the upper or lower jaw region of the mouth, since in this case it is particularly important to precisely determine, by means of the optical scanner system, the relative position and orientation of the dental implants, and to obtain a plaster mold in which the analogs have the exact same relative position and orientation as those of the dental implants. In this case, at least two scan abutments according to the invention are used, and the method comprises at least the following successive steps:
[a] fixing one of the scan abutments to each of the dental implants by coupling the coupling base to said dental implant;
[b] applying a mass of impression material on the upper or lower jaw region of the mouth, such that the retaining projections of each of the scan abutments fixed to the dental implants are plunged into said mass of impression material and the at least one singular area of the scan head of each of said scan abutments is outside said mass of impression material; such that an impression of said upper or lower jaw region of the mouth is formed in said mass of impression material, wherein said scan abutments are made integral with said mass of impression material by said retaining projections;
[c] releasing the scan abutments from the dental abutments and removing the mass of impression material, which comprises said scan abutments integral with the mass of impression material, from the mouth;
and wherein, after step [a], scanning of the scan heads of the scan abutments is performed by means of an optical scanner system which recognizes the at least one singular area; and based on the recognition of said singular areas, a position and an orientation of said scan abutments are determined.

Preferably, the retaining projections are arranged in a plurality of rows separated from one another, along the axis, by transverse bands of the shaft in planes orthogonal to said axis, such that in said transverse bands the outer surface of the shaft is devoid of said retaining projections. This allows the scan abutment to be readily cut at a desired height between two rows of projections and to be used as a fixed abutment for restoration in the mouth.

Preferably, the retaining projections are arranged in a plurality of columns separated from one another, along a circumference centered in the axis, by longitudinal bands of the shaft in planes comprising said axis, such that in said longitudinal bands the outer surface of the shaft is devoid of said retaining projections. In at least several of said longitudinal bands, said outer surface of the shaft comprises a planar face. This planar face is an additional singular area that can be recognized by the optical scanner system, together with the other singular areas located in the scan head, so as to better determine a position and an orientation of the scan abutment when the rod is not covered by the impression material.

Preferably, the outer surface of the shaft comprises a planar face in each of the longitudinal bands. Multiple planar faces acting as additional singular areas, distributed along the perimeter of the rod, are thereby provided, so the optical scanner system will more readily recognize one of these planar faces regardless of the position of the scan abutment in the mouth.

Preferably, at least one of the retaining projections comprises a planar face at a free end of said projection opposite the shaft. This planar face is also an additional singular area that can be recognized by the optical scanner system as the planar face in the longitudinal band that is, however, located in a better position to be recognized by the optical scanner system.

Preferably, each of said retaining projections comprises a planar face at a free end of said retaining projection opposite the shaft. Multiples planar faces acting as additional singular areas, distributed along the perimeter of the rod and located in a better position than the planar faces in the longitudinal bands, are thereby provided.

Preferably, the scan head comprises on its outer surface a plurality of said singular areas, selected from those of the group comprising a planar face in a plane not orthogonal to the axis and a notch. More preferably, at least some of these singular areas consist of notches formed at least partially on an end face of said scan head opposite the rod. These notches allow a particularly effective detection of the singular areas by the optical scanner system, even when the scan head is plunged almost completely into the mass of impression material, but the end face is free of said impression material.

Preferably, the end face of the scan head is planar, and the notches are formed at a perimetral edge of said end face of the scan head. This configuration allows an even more effective detection of the singular areas by the optical scanner system.

Preferably, the notches form an asymmetric assembly with respect to the axis. This allows the optical scanner system to more easily deduce the orientation of the scan abutment.

Embodiments in which the scan abutment is designed for being fixed by pressure to the dental implant are possible. However, in the preferred embodiments, the scan abutment comprises a through hole along the axis for receiving a fixing screw for fixing said scan abutment to the dental implant, and an internal border formed in said through hole to serve as a stop with respect to said fixing screw.

Preferably, the length of the rod in the direction of the axis, between the retaining projection closest to the scan head and the retaining projection farthest away from the scan head in the direction of said axis and including the thickness of these two retaining projections, is greater than or equal to 3 mm; and in a plane orthogonal to the axis, the difference between the radial distance between said axis and the point of a retaining projection farthest away from said axis and the radial distance between said axis and the point of the outer surface of the shaft closest to said axis is greater than or equal to 0.3 mm. On one hand, these dimensions are suitable for facilitating the arrangement of the scan head outside the mass of impression material, and therefore the singular areas can be scanned after step [c], and on the other hand, for the effective retention by the retaining projections of the scan abutment in the mass of impression material.

The invention also relates to the use of a scan abutment according to the invention for creating a model of an upper or lower jaw region of a mouth, wherein the same scan abutment is used as an impression coping, plunged into a mass of impression material with the exception of at least one of the one or more singular areas not covered by the impression material, and as a scan abutment which is scanned by an optical scanner system which recognizes said at least one singular area and which, based on the recognition of said at least one singular area, determines a position and an orientation of said scan abutment.

The invention also comprises other detailed features shown in the following detailed description of an embodiment of the invention and in the accompanying figures.

### Brief description of the drawings

The advantages and features of the invention are apparent from the following description in which a preferred embodiment of the invention is described in a non-limiting manner with respect to the scope of the main claim in reference to the figures.
Figures 1 to 12 show the scan abutment. Figure 1 is a top perspective view. Figure 2 is a top perspective view from another angle. Figure 3 is a bottom perspective view. Figure 4 is a front view. Figure 5 is a view from the right side. Figure 6 is a view from the left side. Figure 7 is a rear view. Figure 8 is a top view. Figure 9 is a bottom view. Figure 10 is a view sectioned along a transverse segment. Figure 11 is a top perspective view sectioned along a longitudinal plane. Figure 12 is a bottom perspective view sectioned along the same longitudinal plane.
Figures 13 and 15 are perspective views of a fixing screw which is used for fixing the scan abutment to the dental implant.
Figures 15 and 16 are perspective views showing the scan abutment with the fixing screw introduced therein.
Figure 17 is a perspective view showing an upper jaw region of a mouth, scan abutments depicted facing the corresponding dental implants implanted in said upper jaw region, and fixing screws depicted facing the scan abutments.
Figure 18 is the same perspective view with the scan abutments fixed to the dental implants by means of fixing screws.
Figures 19 and 20 are a bottom perspective view and a top perspective view of a tray, respectively.
Figure 21 is the same perspective view as in Figures 17-18, with the tray placed against the upper jaw region after having been refilled with an impression material.
Figure 22 is a perspective view showing the integral assembly formed by the tray, the mass of impression material, and the scan abutments held in said mass, after said integral assembly has been removed from the upper jaw region. The figure shows an optical scanner in the position for scanning the face of the integral assembly through which the scan heads protrude.
Figure 23 is a perspective view showing the same integral assembly as Figure 22, but through the impression face, i.e., the face that was in contact with the upper jaw region and in which an impression of said upper jaw region has been obtained. The figure shows the optical scanner in the position for scanning this impression face.

### Detailed description of an embodiment of the invention

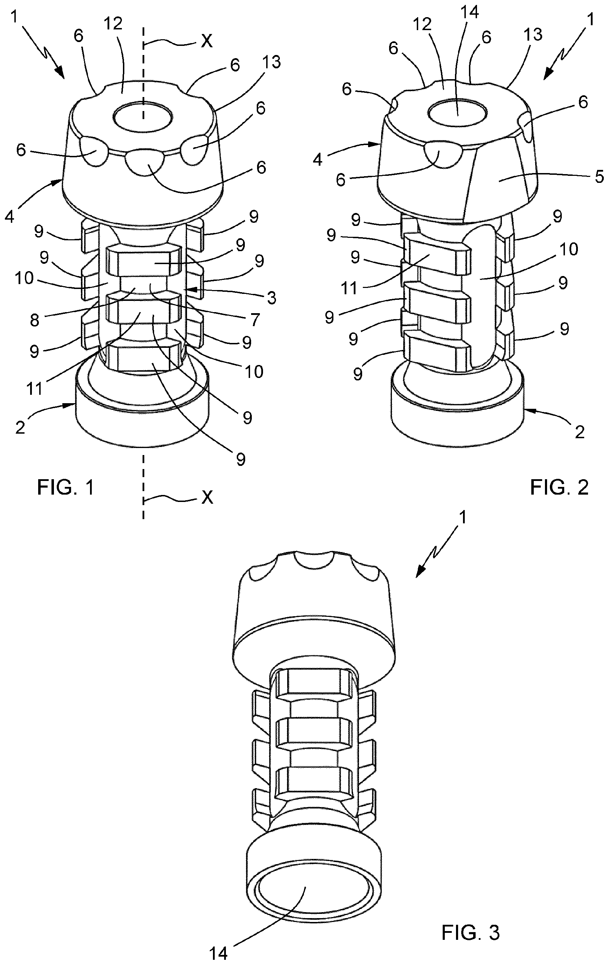

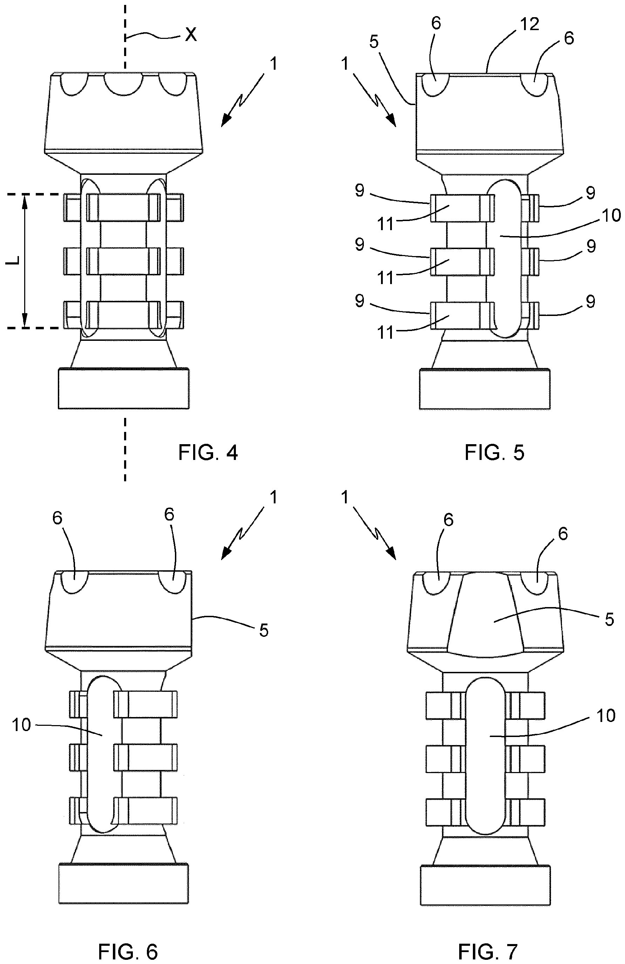

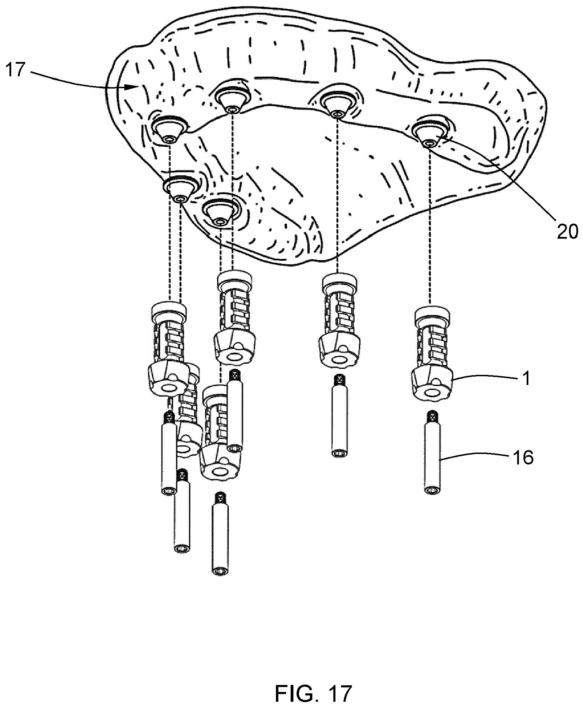

Figures 1 to 12 show an embodiment of the scan abutment 1 for dental implants according to the invention. The scan abutment 1 is a monoblock piece, in this case a piece made of titanium with an alumina oxide coating, but it can also be made from any other suitable material. It is in the shape of an elongated abutment with a coupling base 2 for coupling the scan abutment 1, directly or through an intermediate piece, to the dental implant, a rod 3 extending from the coupling base 2 along a longitudinal axis X, and a scan head 4 formed at one end of the rod 3 opposite the coupling base 2. In the depicted example, the coupling base is formed for being indirectly coupled to a dental implant by means of an intermediate piece which, in this case, is a transepithelial abutment 20 which is coupled to a dental implant implanted in an upper jaw bone or a lower jaw bone of a mouth. The transepithelial abutment 20 protruding from the mucous tissue of an upper jaw region 17 of a mouth can be seen in Figure 17.

The scan head 4 has an almost cylindrical, slightly frustoconical shape with a planar end face 12 opposite the rod 3. In the depicted example, this planar end face 12 is in a plane orthogonal to axis X. A through hole 14, coaxial with axis X, which goes through the entire scan abutment 1 and is intended for receiving a fixing screw 16 for fixing the scan abutment 1 to the dental implant passes through the center of this end face 12.

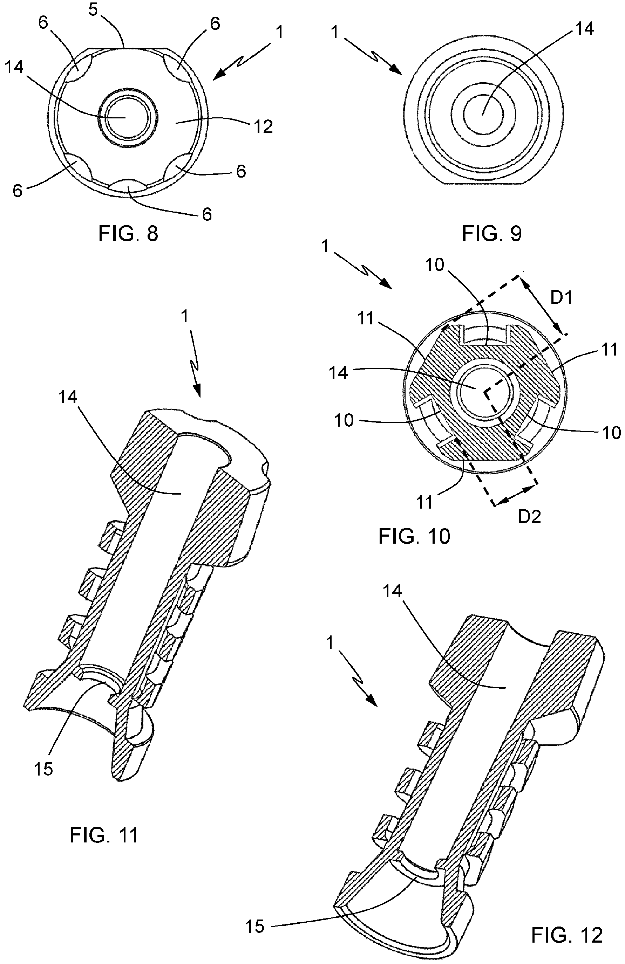

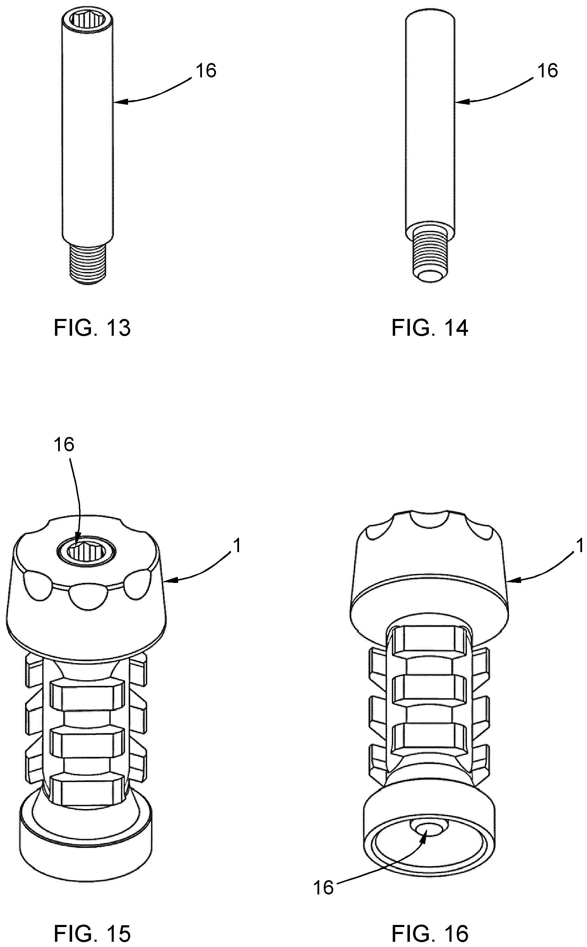

The fixing screw 16 is depicted in Figures 13 and 14. As can be seen in Figures 11 and 12, the through hole 14 has an internal border 15 formed therein which serves as a stop for the fixing screw 16. Figures 15 and 16 show the scan abutment 1 with the fixing screw 16 introduced in the through hole 14.

Singular areas 5, 6 which can be recognized by an optical scanner system for determining a position and an orientation of the scan abutment 1 are formed on the outer surface of the scan head 4. In the depicted example, these singular areas 5, 6 consist of a planar face 5 and notches 6. The planar face 5 is formed in the side wall of the scan head 4 and is in a plane parallel to axis X. In this case, there are five notches 6 arranged in two groups: a first group of three notches 6 together, in a position opposite the planar face 5 with respect to axis X, and a second group of two notches 6 each arranged on one side of the planar face 5. These notches 6 thereby form an asymmetric assembly with respect to axis X. The notches 6 are formed at a perimetral edge 13 of the end face 12 of the scan head 4, such that they occupy a perimetral part of said end face 12.

The rod 3 comprises a shaft 7 with an outer surface 8 and retaining projections 9 extending from said shaft 7, radially with respect to axis X, from said outer surface 8 of the shaft 7. The outer surface 8 of the shaft 7 is cylindrical and has recesses forming planar faces 10 parallel to axis X. There are nine retaining projections 9 arranged in three rows separated from one another, along axis X, by transverse bands of the shaft 7 in planes orthogonal to axis X, such that in said transverse bands the outer surface 8 of the shaft 7 is devoid of retaining projections 9. There are three retaining projections 9 in each row, regularly arranged around axis X. Furthermore, the retaining projections 9 are arranged in three columns separated from one another, along a circumference centered in axis X, by longitudinal bands of the shaft 7 in planes comprising axis X, such that in said longitudinal bands the outer surface 8 of the shaft 7 is devoid of retaining projections 9. In each of these longitudinal bands, the outer surface 8 of the shaft 7 comprises a planar face 10 formed by a recess in the cylindrical shape of said outer surface 8. All the retaining projections 9 are substantially equal and have a disk shape orthogonal to axis X, recessed at a free end opposite the shaft for a planar face 11 at said end.

With reference to Figure 10, the length L is greater than or equal to 3 mm. The length L is the length of the rod 3 in the direction of axis X, between the retaining projection 9 closest to the scan head 4 and the retaining projection 9 farthest away from said scan head 4 in the direction of axis X and including the thickness of these two retaining projections 9, and it is greater than or equal to 3 mm. The difference between D1 and D2 is greater than or equal to 0.3 mm. D1 is the radial distance, in a plane orthogonal to axis X, between axis X and the point of a retaining projection 9 farthest away from axis X. D2 is the radial distance, in a plane orthogonal to axis X, between axis X and the point of the outer surface 8 of the shaft 7 closest to axis X. In the depicted example, the total length of the scan abutment 1 along axis X is 12.5 mm. The length L is 5 mm. The thickness of the retaining projections 9, in the direction of axis X, is 1 mm. The separation between retaining projections 9, in the direction of axis X, is 1 mm. The distance D1 is 2.2 mm and corresponds to the radius of the disks forming the retaining projections. The distance D2 is 1.4 mm and corresponds to the distance between the planar face 10 and axis X. The difference between D1 and D2 is 0.8 mm.

A method according to the invention for creating a model of an upper or lower jaw region of a mouth using the scan abutments 1 is illustrated in Figures 17 to 23. These figures depict an upper jaw region 17 of a mouth having six dental implants. In this example, the upper jaw region 17 is without teeth, but in other examples it can have teeth next to the dental implants. Transepithelial abutments 20 which are coupled to the dental implants (not seen in the figures) implanted in the bone of the upper jaw region 17 have been depicted. Other embodiments with intermediate pieces of different shapes, instead of the depicted transepithelial abutments 20, or without any intermediate piece, such that the scan abutment 1 is coupled directly to the dental implant, are possible. The only difference is that the coupling base 2 of the scan abutment 1 will have a shape suitable for being coupled to the interposed piece or to the dental implant.

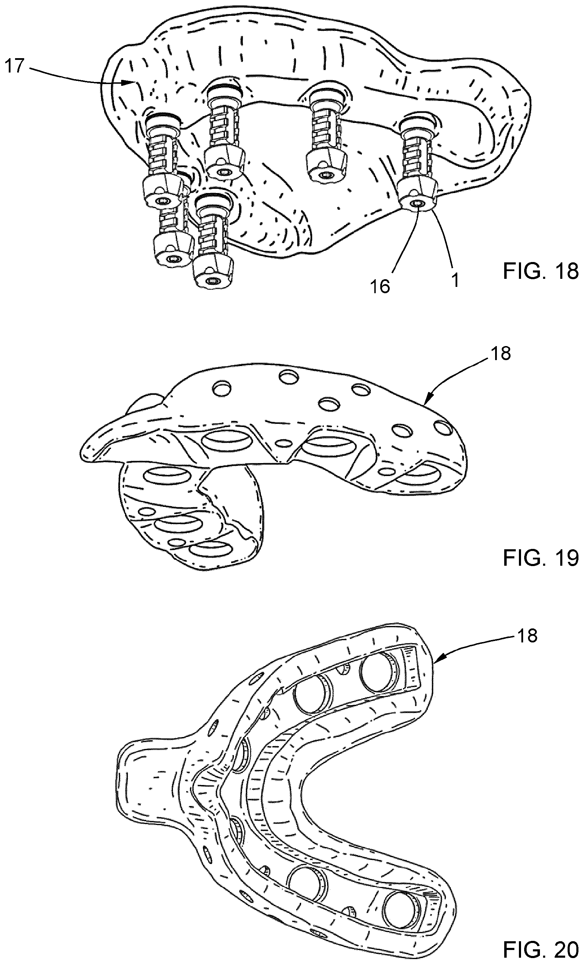

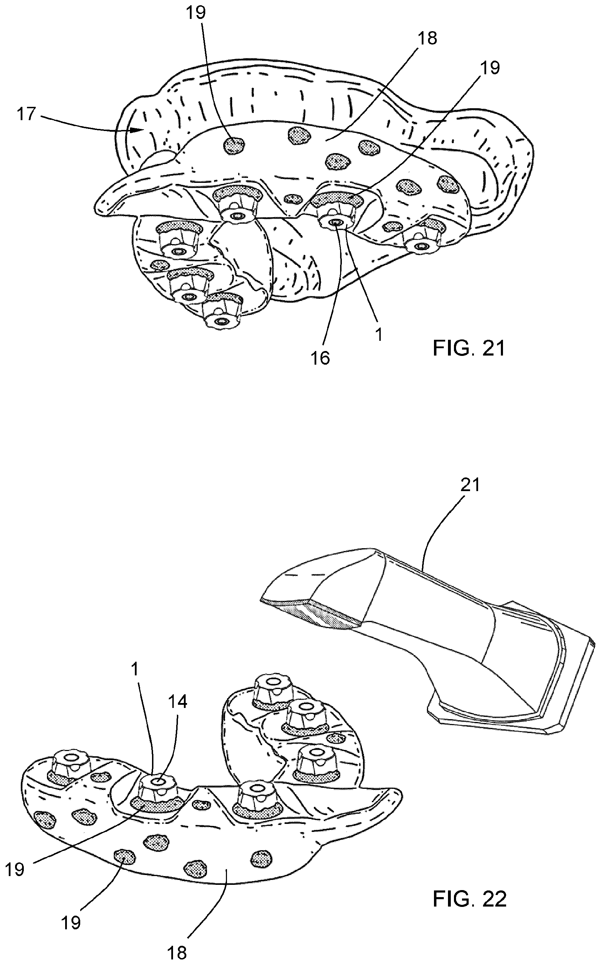

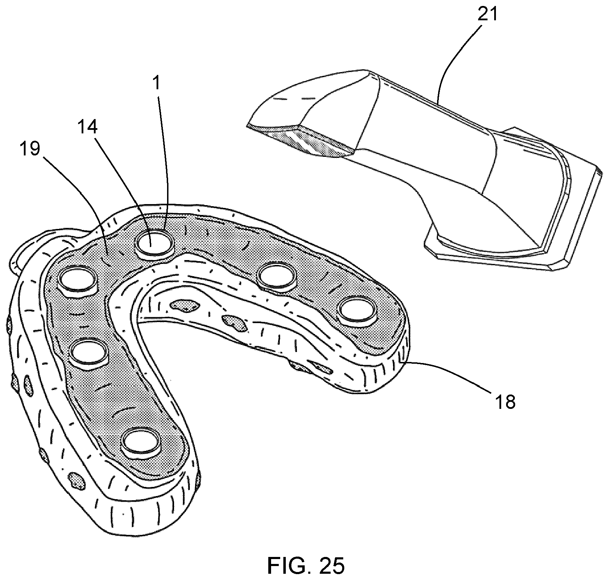

The method has the steps following:
[a] A scan abutment 1 is fixed to each of the dental implants by coupling the coupling base 2 to the dental implant through the transepithelial abutment 20 which is in turn coupled to the dental implant. To that end, there is used a fixing screw 16 which passes through the through hole 14 of the scan abutment, goes through a corresponding through hole of the transepithelial abutment 20, and is screwed into the dental implant. Figures 17 and 18 show the state before and after fixing the scan abutments 1 to the dental implants, respectively.
[b] A mass of impression material 19 is applied on the upper jaw region 17 comprising the scan abutments 1 fixed to the dental implants, such that the retaining projections 9 of each of the scan abutments 1 fixed to the dental implants are plunged into said mass of impression material 19, and the singular areas 5, 6 of the scan head 4, preferably all of them or at least the notches 6, of each of the scan abutments 1 are outside the mass of impression material 19. An impression of the upper jaw region is formed in the mass of impression material 19, and the scan abutments 1 are made integral with the mass of impression material 19 by the retaining projections 9. To that end, an open tray 18 like the one shown in Figures 19 and 20 is used, for example. The tray 18 has openings for the passage of the scan heads 4 and holes for allowing the exit of excess impression material 19. The tray 18 is filled with a mass of impression material 19 and then applied with force against the upper jaw region 17, such that the scan heads 4 protrude through the openings of the tray 18 and excess impression material 19 exits through the holes. A little excess impression material 19 also exits through each opening of the tray 19 around the impression head 4. As a non-limiting example, alginate or silicone can be used as an impression material 18. Figure 21 shows the state at the end of this step [b].
[c] Once the impression material 19 has hardened, all the screws 16 are taken out to release all the scan abutments 1 from the dental abutments. The integral assembly formed by the tray 18, the hardened mass of impression material 19, and the scan abutments 1 integral with said mass of impression material 19, is then removed from the mouth. This integral assembly which has been removed from the mouth is shown in Figures 22 and 23.

After step [c], said integral assembly is scanned with an optical scanner 21. As shown in Figure 22, a first scanning is performed on the face where the scan heads 4 of the scan abutments 1 protrude, such that all the scan heads 4 are scanned. The optical scanner 21 is part of a system comprising software which is executed in a processor. The software receives the signal or data from the optical scanner 21, recognizes the singular areas 5, 6, and based on the recognition of said singular areas 5, 6, determines a position and an orientation of each of the scan abutments 1. As shown in Figure 23, said integral assembly can also be scanned with the optical scanner 21 on the impression face, i.e., the face where the mass of impression material 19 has the impression of the upper jaw region. The software thereby generates a CAD file which defines the surface of the mucous tissue of the upper jaw region which has been impressed in the mass of impression material 19.

Once this scanning is performed, a model made of plaster or of another suitable material is made using the impression obtained in the mass of impression material 19 as a mold. This plaster mold accurately reproduces the surface of the upper jaw region and contains analogs having the same position and orientation as the dental implants. This process is not described herein, since it is known to one skilled in the art.

## Claims

1. A scan abutment (1) for dental implants suitable for being removably fixed to a dental implant implanted in an upper jaw bone or a lower jaw bone of a mouth, said scan abutment (1) being a monoblock piece comprising a coupling base (2) for coupling said scan abutment (1), directly or through an intermediate piece, to the dental implant, a rod (3) extending from said coupling base (2) along an axis (X), and a scan head (4) formed at one end of said rod (3) opposite said coupling base (2), said scan head (4) comprising on its outer surface at least one singular area (5, 6) that can be recognized by an optical scanner system to determine a position and an orientation of said scan abutment (1), **characterized in that** said rod (3) comprises a shaft (7) with an outer surface (8) and retaining projections (9) extending from said shaft (7), radially with respect to said axis (X), from said outer surface (8) of the shaft (7).

2. The scan abutment (1) according to claim 1, **characterized in that** said retaining projections (9) are arranged in a plurality of rows separated from one another, along said axis (X), by transverse bands of said shaft (7) in planes orthogonal to said axis (X), such that in said transverse bands said outer surface (8) of the shaft (7) is devoid of said retaining projections (9).

3. The scan abutment (1) according to any one of claims 1 or 2, **characterized in that** said retaining projections (9) are arranged in a plurality of columns separated from one another, along a circumference centered in said axis (X), by longitudinal bands of said shaft (7) in planes comprising said axis (X), such that in said longitudinal bands said outer surface (8) of the shaft (7) is devoid of said retaining projections (9); and **in that**, in at least several of said longitudinal bands, said outer surface (8) of the shaft (7) comprises a planar face (10).

4. The scan abutment (1) according to claim 3, **characterized in that** said outer surface (8) of the shaft (7) comprises a planar face (10) in each of said longitudinal bands.

5. The scan abutment (1) according to any one of claims 1 to 4, **characterized in that** at least one of said retaining projections (9) comprises a planar face (11) at a free end of said retaining projection (9) opposite said shaft (7).

6. The scan abutment (1) according to claim 5, **characterized in that** each of said retaining projections (9) comprises a planar face (11) at a free end of said retaining projection (9) opposite said shaft (7).

7. The scan abutment (1) according to any one of claims 1 to 6, **characterized in that** said scan head (4) comprises on its outer surface a plurality of said singular areas (5, 6), selected from those of the group comprising a planar face (5) in a plane not orthogonal to said axis (X) and a notch (6).

8. The scan abutment (1) according to any one of claims 1 to 8, **characterized in that** said scan head (4) comprises on its outer surface a plurality of said singular areas (5, 6) at least some of which consist of notches (6) formed at least partially on an end face (12) of said scan head (4) opposite said rod (3).

9. The scan abutment (1) according to claim 8, **characterized in that** said end face (12) of the scan head (4) is planar and said notches (6) are formed at a perimetral edge (13) of said end face (12) of the scan head (4).

10. The scan abutment (1) according to any one of claims 8 or 9, **characterized in that** said notches (6) form an asymmetric assembly with respect to said axis (X).

11. The scan abutment (1) according to any one of claims 1 to 10, **characterized in that** it comprises a through hole (14) along said axis (X) for receiving a fixing screw, for fixing said scan abutment (1) to the dental implant, and an internal border (15) formed in said through hole (14) to serve as a stop with respect to said fixing screw.

12. The scan abutment (1) according to any one of claims 1 to 11, **characterized in that** the length (L) of said rod (3) in the direction of said axis (X), between the retaining projection (9) closest to said scan head (4) and the retaining projection (9) farthest away from said scan head (4) in the direction of said axis (X) and including the thickness of these two retaining projections (9), is greater than or equal to 3 mm; and in a plane orthogonal to said axis (X), the difference between the radial distance (D1) between said axis (X) and the point of a retaining projection (9) farthest away from said axis (X) and the radial distance (D2) between said axis (X) and the point of the outer surface (8) of the shaft (7) closest to said axis (X) is greater than or equal to 0.3 mm.

13. A method for creating a model of an upper or lower jaw region of a mouth using at least one scan abutment (1) according to any one of claims 1 to 12, wherein at least one dental implant is implanted in a bone of said upper or lower jaw region of the mouth, said method comprising at least the following successive steps:
[a] fixing said scan abutment (1) to said dental implant by coupling said coupling base (2) to said dental implant;
[b] applying a mass of impression material on said upper or lower jaw region of the mouth, such that said retaining projections (9) of the scan abutment (1) fixed to the dental implant are plunged into said mass of impression material and said at least one singular area (5, 6) of the scan head (4) of said scan abutment (1) is outside said mass of impression material; such that an impression of said upper or lower jaw region of the mouth is formed in said mass of impression material, wherein said scan abutment (1) is made integral with said mass of impression material by said retaining projections (9);
[c] releasing said scan abutment (1) from said dental implant and removing said mass of impression material, which comprises said scan abutment (1) integral with said mass of impression material, from said mouth;
and wherein after step [a], preferably after step [c], scanning of said scan head (4) of the scan abutment (1) is performed by means of an optical scanner system which recognizes said at least one singular area (5, 6); and based on the recognition of said singular areas (5, 6), a position and an orientation of said scan abutment (1) are determined.

14. The method according to claim 13, **characterized in that** at least two scan abutments (1) according to any one of claims 1 to 12 are used, wherein at least two dental implants are implanted in a bone of said upper or lower jaw region of the mouth, said method comprising at least the following successive steps:
[a] fixing one of said scan abutments (1) to each of said dental implants by coupling said coupling base (2) to said dental implant;
[b] applying a mass of impression material on said upper or lower jaw region of the mouth, such that said retaining projections (9) of each of said scan abutments (1) fixed to said dental implants are plunged into said mass of impression material and said at least one singular area (5, 6) of the scan head (4) of each of said scan abutments (1) is outside said mass of impression material; such that an impression of said upper or lower jaw region of the mouth is formed in said mass of impression material, wherein said scan abutments (1) are made integral with said mass of impression material by said retaining projections (9);
[c] releasing said scan abutments (1) from said dental abutments and removing said mass of impression material, which comprises said scan abutments (1) integral with said mass of impression material, from said mouth;
and wherein after step [a], preferably after step [c], scanning of said scan heads (4) of the scan abutments (1) is performed by means of an optical scanner system which recognizes said singular areas (5, 6); and based on the recognition of said singular areas (5, 6), a position and an orientation of said scan abutments (1) are determined.

15. Use of a scan abutment (1) according to any one of claims 1 to 12 for creating a model of an upper or lower jaw region of a mouth, wherein the same scan abutment (1) is used as an impression coping, plunged into a mass of impression material with the exception of at least one of said one or more singular areas (5, 6) not covered by the impression material, and as a scan abutment which is scanned by an optical scanner system which recognizes said at least one singular area (5, 6) and which, based on the recognition of said at least one singular area (5, 6), determines a position and an orientation of said scan abutment (1).
